# DEMANDE DE BREVET EUROPEEN

(11) **EP 1 269 941 A1**
(43) Date de publication de la demande: **02.01.2003**
(21) Numéro de dépôt: 02291532.6
(22) Date de dépôt: 19.06.2002
(51) Int. Cl.: A61F 2/40, A61F 2/46

(54) **Prothèse d'épaule**

(30) Priorité: 21.06.2001 FR 0108207
(71) Demandeur: Depuy France, 69800 Saint Priest (FR)
(72) Inventeur: Capon, Didier, 44880 Sautron (FR); Delince, Phillipe, 1380 Lasne (BE); De Wilde, Lieven, 9000 Gand (BE); Desmoineaux, Pierre, 78150 Le Chesnay (FR); Dumontier, Philippe, 03400 Yzeure (FR); Katz, Denis, 56270 Ploemeur (FR); Lesur, Etienne, 54290 Bayon (FR); Oudet, Didier, 37540 St Cyr sur Loire (FR); Godet, Anne-Céline, 69008 Lyon (FR)
(74) Mandataire: Bernasconi, Jean Raymond

(57) **Abrégé**

L'invention a pour objet une prothèse d'épaule (5, 32), comportant une tige humérale (9, 39) destinée à être introduite dans le canal médullaire d'un humérus fracturé (2) et une tête (12), sensiblement hémisphérique susceptible d'être rendue solidaire de la tige humérale et adaptée pour venir se loger dans une glène de l'épaule, caractérisée en ce que la tête humérale (12), peut être fixée sur la tige humérale dans un plan radial quelconque par rapport à l'axe de la tige humérale (9, 39), après fixation de la tige (9, 39) dans l'humérus, et fixation des muscles de l'épaule sur la tête.

## Description

La présente invention a pour objet une prothèse d'épaule, comportant une tige humérale destinée à être introduite dans le canal médullaire d'un humérus fracturé, une partie métaphysaire solidarisée avec la tige et une tête sensiblement hémisphérique solidaire de la partie métaphysaire, adaptée pour venir se loger dans une glène de l'épaule.

On sait que, par suite d'une fracture, l'extrémité supérieure d'un humérus peut être fracturée en plusieurs fragments (quatre et davantage).

Les problèmes posés par une telle fracture multi-fragmentaire étaient particulièrement difficiles à résoudre. Cependant, le document EP 0940126 a décrit un moyen extrêmement efficace qui permet de fixer les tubérosités de la coiffe sur une tête humérale de prothèse dans des conditions autorisant un rétablissement naturel de la rétroversion de la tête et rétablissant une tension normale des différents éléments de la coiffe, tout en autorisant un rétablissement de la longueur du bras.

Le document EP 0940126 décrit ainsi une prothèse d'épaule dans laquelle la tête humérale est solidaire d'un axe introduit dans un alésage de la partie métaphysaire, incliné sur l'axe longitudinal de cette dernière. Une vis pourvue d'un téton, vissée axialement dans la partie métaphysaire, vient bloquer en place l'axe solidaire de la tête par introduction du téton dans une encoche de l'axe. Pour mettre en place cette prothèse, on fixe d'abord les muscles sur la tête, puis on monte la tête sur la tige, laquelle est ensuite bloquée par la vis.

En fait, le chirurgien rencontre de sérieuses difficultés dans ce processus, car il doit placer la tête sur la tige humérale à partir du dessus du champ opératoire, sur lequel il ne possède pas de visibilité. En outre, compte tenu de la position de la tête par rapport à l'extrémité de la partie métaphysaire, la vis de blocage est très difficile à mettre en place. Enfin, cet agencement présente l'inconvénient de concentrer tous les efforts sur le téton de blocage de la vis, ce qui fragilise notablement la prothèse.

En définitive, une prothèse d'épaule du type défini ci-dessus doit pouvoir satisfaire à un certains nombres d'impératifs :
. présenter une morphologie épiphysaire et métaphysaire permettant la reconstruction anatomique de « l'offset », du débord postérieur et de la rétrotorsion de l'extrémité supérieure de l'humérus,
. présenter une morphologie autorisant la reconstruction et le maintien anatomique des tubérosités humérales,
. présenter la possibilité, pour tout ou partie des pièces la constituant, de refixer la coiffe des rotateurs et de maintenir dans le temps la consolidation des tubérosités.

Pour permettre une reconstruction en hauteur convenable de l'humérus, la tige de la prothèse doit être correctement positionnée en hauteur, c'est à dire qu'elle ne doit être placée ni trop haute ni trop basse.

On a déjà proposé, dans les documents FR 2579454 et 2699400, des prothèses d'épaule dans lesquelles la tête humérale peut être fixée sur la tige humérale dans un plan radial quelconque par rapport à l'axe de la tige humérale pour compenser le fait que les tiges humérales de ces documents sont fixées dans l'humérus dans une position angulaire aléatoire, mais ces prothèses ne sont pas adaptées au problème résolu par l'invention.

Conformément à la présente invention, la prothèse comporte, d'une part, une tête humérale, et, d'autre part, une tige humérale destinée à être fixée axialement dans l'humérus, et est caractérisée en ce que la tête humérale peut être fixée sur la tige humérale dans un plan radial quelconque par rapport à l'axe de la tige humérale, après fixation de la tige humérale dans l'humérus.

De préférence, la tête humérale est solidaire, par exemple en venant de moulage, d'une partie métaphysaire qui est susceptible d'être assemblée à la tige humérale dans une position angulaire quelconque.

De préférence, les moyens de fixation sur la tige humérale comportent un assemblage d'éléments mâle et femelle, permettant une fixation dans n'importe quelle position angulaire relative.

Par n'importe quelle position angulaire relative on entend, de préférence, un assemblage axial, c'est-à-dire dans l'axe de la tige humérale avec des moyens de fixation permettant une fixation dans toute position angulaire autour de cet axe. Cependant, de façon moins préférée, on peut prévoir que les positions angulaires soient déterminées par des moyens d'indexation, pourvu que l'angle entre deux moyens d'indexation consécutifs soit faible.

Il est ainsi possible, une fois que la tête humérale a été fixée aux différents éléments de la coiffe des rotateurs, d'autoriser la tête à prendre sa position angulaire naturelle puis, la tige ayant été fixée dans l'humérus, d'assurer la solidarisation, quelle que soit la position angulaire relative de la tête humérale et de la tige.

De façon particulièrement préférée, la tête humérale comprend des moyens de fixation des éléments de la coiffe des rotateurs, tels que définis dans la demande européenne EP-0940126.

Ces moyens peuvent être répartis en périphérie de la surface articulaire de la tête prothétique, par exemple sous forme de trous, permettant la fixation des éléments de muscles de l'épaule tandis que les tubérosités ou leurs fragments peuvent être fixées en-dessous sur la tête.

Ainsi la prothèse peut être réalisée en au moins deux parties, à savoir la tige humérale d'une part, la partie métaphysaire et la tête humérale d'autre part, l'assemblage de ces deux parties pouvant être réalisé au moyen d'un système mâle-femelle, par exemple du type cône morse.

Suivant un mode de réalisation de l'invention, la partie métaphysaire délimite une partie femelle sous la forme d'une douille conique terminale tandis que l'extrémité proximale associée de la tige est constituée d'une partie conique mâle complémentaire, adaptée pour être introduite dans la douille, ces deux parties étant coaxiales à l'axe longitudinal de la tige.

Suivant une caractéristique complémentaire, les moyens de fixation comprennent une vis adaptée pour être vissée dans un trou taraudé correspondant ménagé axialement dans la partie conique mâle, des moyens étant prévus pour permettre l'introduction de la vis dans ledit trou taraudé.

Pour mettre en place cette prothèse, le chirurgien positionne d'abord la tige humérale à la hauteur adéquate dans le canal médullaire de l'humérus puis la scelle dans l'humérus. Puis il fixe les muscles de l'épaule, en particulier la coiffe des rotateurs, sur la tête, au moyen de fils. Ensuite, le chirurgien vient positionner les parties d'os fracturées sur la tête. Puis il monte la tête humérale sur la tige déjà scellée, sans la fixer définitivement, pour pouvoir effectuer les réglages de rétroversion. Ensuite, il fixe définitivement, par impaction, la tête sur la tige. Enfin, il lace les fragments osseux sur la tête et, éventuellement, tend les fils de fixation de la coiffe sur la tête.

On comprend qu'il peut ainsi obtenir, comme décrit dans la demande européenne précitée, une position de rétroversion idéale de la tête humérale et une position angulaire de la tige humérale dans l'humérus optimale pour la fixation de cette tige, laquelle peut d'ailleurs posséder une surface extérieure, et notamment vers la métaphyse qui n'est pas de révolution, et qui peut être très adaptée au canal médullaire.

En conséquence, on obtient facilement un positionnement angulaire parfait de la tête humérale dans sa position anatomique tenant compte des muscles de la coiffe et on réduit, ou supprime, la sollicitation s'exerçant sur les tubérosités fracturées, qui peuvent beaucoup plus facilement se lier, notamment par croissance osseuse, sur la tête prothétique, au-dessous de la surface articulaire.

L'invention a également pour objet l'utilisation d'un ensemble d'une tige humérale et d'une tête humérale pouvant être fixée sur la tige dans un plan radial quelconque par rapport à l'axe de la tige humérale, après fixation de la tige humérale dans l'humérus, pour la fabrication d'une prothèse munie de moyens de fixation de fragments osseux sur la périphérie de la tête, et destinée au traitement prothétique des factures multi-fragmentaires de l'extrémité supérieure de l'humérus.

L'invention a également pour objet un jeu de prothèses contenant une pluralité de prothèses humérales de dimensions différentes et/ou de prothèses de tête humérale de dimensions différentes.

De préférence on utilise des prothèses d'essai possédant une tête humérale et une cale axiale destinées à venir en contact avec un bord de la fracture humérale et l'on scelle la tige humérale dans la position déterminée par la cale de la prothèse d'essai.

L'invention a également pour objet les prothèses d'essai ainsi définies ainsi qu'un jeu de ces prothèses d'essai.

D'autres particularités et avantages de l'invention apparaîtront au cours de la description qui va suivre, faite en référence aux dessins annexés qui en illustrent plusieurs formes de réalisation à titre d'exemples non limitatifs.

La figure 1 est une vue en élévation frontale simplifiée de l'extrémité supérieure fracturée d'un humérus.

La figure 2 est une vue en coupe longitudinale partielle éclatée, d'une première forme de réalisation de la prothèse d'épaule conforme à l'invention.

La figure 3 est une vue en plan suivant la flèche 3 de la partie métaphysaire et de la tête de la prothèse de la figure 2.

La figure 4 est une vue en coupe transversale à échelle agrandie par rapport aux figures précédentes, d'une seconde forme de réalisation de la prothèse conforme à l'invention, la coupe étant réalisée au niveau de l'assemblage de l'extrémité de la tige humérale et de la partie métaphysaire.

La Figure 5 est une vue en coupe longitudinale d'une troisième forme de réalisation de la prothèse de l'invention.

La Figure 6 est une vue d'une prothèse d'essai de l'invention.

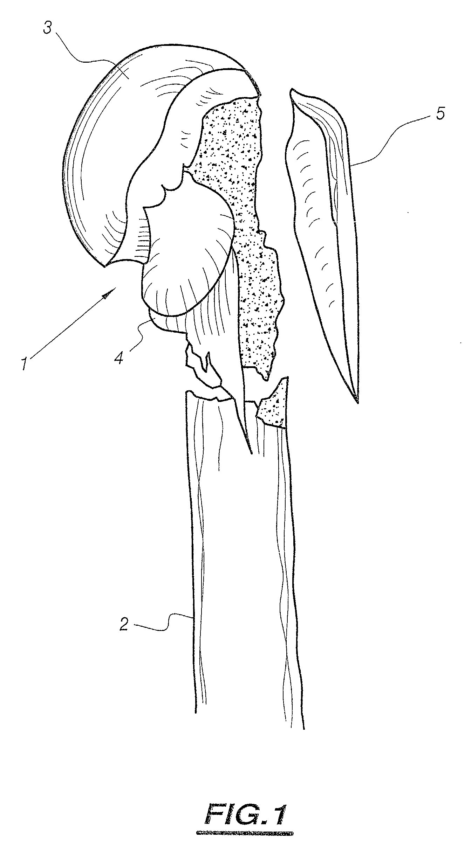

On voit à la figure 1 l'extrémité supérieure 1 fracturée d'un humérus 2, avec la tête humérale 3 détachée et déplacée par rapport au reste de l'humérus, ainsi que par rapport aux tubérosités 4 et 5, à la cavité glénoïde et à l'omoplate.

La prothèse humérale visée par l'invention a pour fonction de permettre la reconstruction en hauteur de l'humérus 2 et la réinsertion de son extrémité dans la cavité glénoïde en recoiffant la tête 3 par les divers muscles environnant, non représentés.

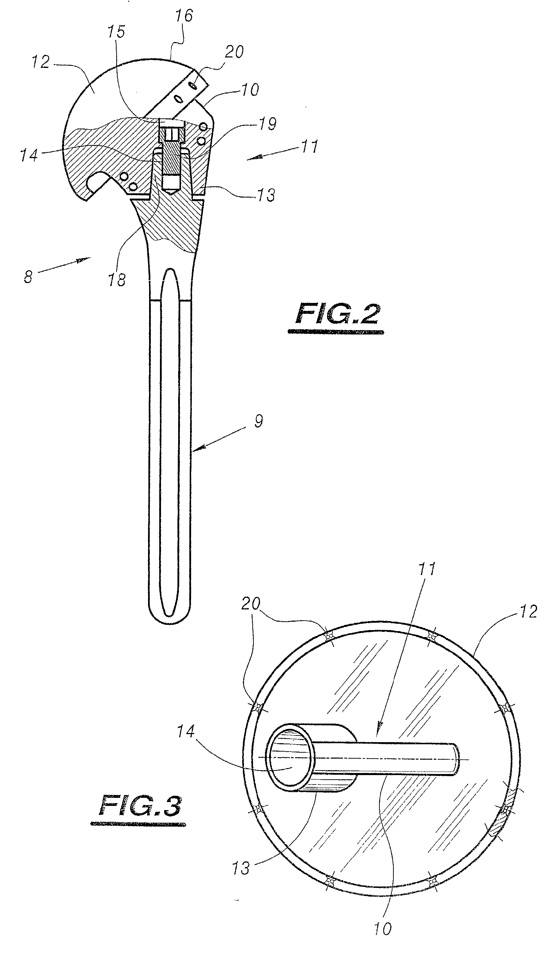

Dans sa première forme de réalisation (figures 2 et 3), la prothèse d'épaule 8 comprend une tige humérale 9 destinée à être introduite dans le canal médullaire de l'humérus fracturé 2, une partie métaphysaire 11 et une partie épyphysaire 12. Cette dernière est constituée par une tête sensiblement hémisphérique adaptée pour venir se loger dans la glène. La tige humérale 9 est solidarisée avec la partie métaphysaire 11 par un assemblage d'éléments mâle et femelle complété par un système de fixation mutuelle desdits éléments, la partie métaphysaire 11 étant de son côté réalisé monobloc avec la tête 12.

Plus précisément, dans le mode de réalisation représenté, la partie métaphysaire 11 comprend un aileron 10 s'étendant dans un plan diamétral de la tête 12 et une partie femelle 13 sous la forme d'une douille conique terminale venue de matière avec l'aileron 10. La douille 13 délimite intérieurement un cône femelle 14 dont le plus grand diamètre est situé à l'opposé de la tête 12. Ce cône femelle 14 se prolonge axialement par un trou lisse cylindrique débouchant lui-même dans un trou de diamètre supérieur 15 de façon à former un épaulement transversal annulaire autour du débouché dans le trou 15. Le trou 15 débouche librement dans la partie supérieure 16, non articulaire, de la tête 12.

L'extrémité proximale 18 associée de la tige 9 est constituée par une partie conique mâle complémentaire de la partie conique femelle 14, et pouvant donc être introduite dans la douille 13.

Les moyens de fixation l'une à l'autre de la partie mâle 18 et de la partie femelle 13 comprennent une vis 19 dont la tige filetée est adaptée pour pouvoir être vissée dans un trou taraudé correspondant ménagé axialement dans la partie conique mâle 18. La vis 19 comporte une tête susceptible d'être vissée par un outil introduit dans le trou 15 pour buter contre l'épaulement du trou 15 et serrer les portées coniques entre elles.

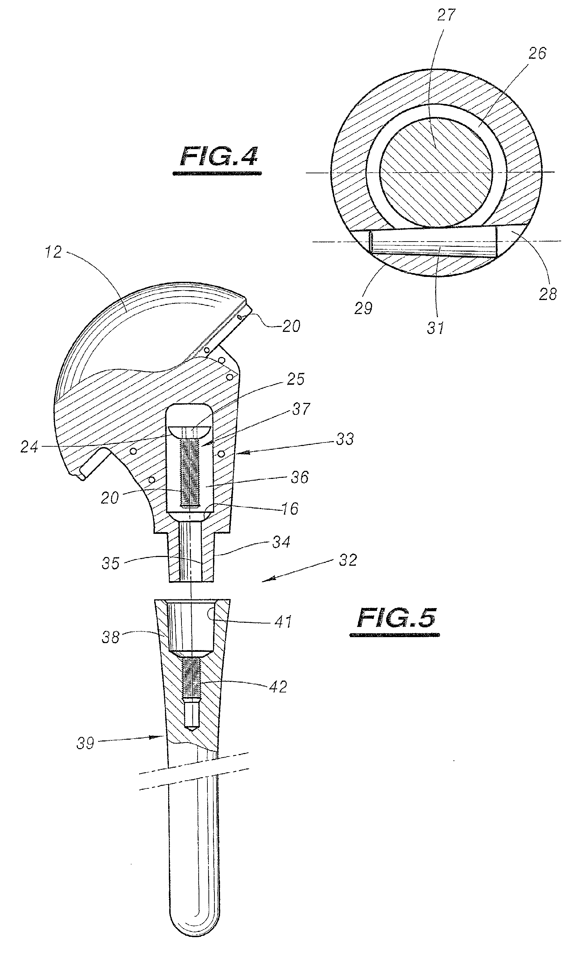

Dans le second mode de réalisation de la prothèse illustré à la figure 4, une gorge circulaire 26 est agencée autour de la partie mâle conique 27, coaxialement à celle-ci. En outre, un alésage transversal conique 28 est réalisé dans la partie femelle correspondante 29 et débouche dans la gorge 26 en la tangentant sensiblement. L'alésage 28 traverse de part en part la partie femelle 29, et est adapté pour recevoir une goupille conique 31 qui peut venir se placer dans la position illustrée à la figure 4, dans laquelle elle traverse la gorge 26. Dans cette position, la goupille 31 réalise un blocage mutuel des parties femelle 29 et mâle 27.

Il convient toutefois d'observer que la mise en place d'une prothèse conforme à la figure 4 peut soulever des difficultés, car la tige humérale 9 est entièrement enfoncée dans l'humérus fracturé 2, de sorte que la goupille 31 devrait d'abord traverser des tissus.

Dans la troisième forme de réalisation illustrée à la figure 5, la prothèse d'épaule 32 comprend une partie métaphysaire 33 toujours réalisée monobloc avec la tête 12, mais qui délimite une partie mâle conique 34 dans laquelle est agencé un alésage lisse 35. Ce dernier débouche dans une ouverture allongée 36, ménagée dans la plaque constitutive de la partie métaphysaire 33 et ayant une longueur légèrement supérieure à la longueur de la vis de blocage 37.

L'extrémité proximale 38 de la tige humérale 39 est constituée d'une partie femelle formant un logement conique 41 adapté pour recevoir la partie mâle 38. Les parties mâle 34 et femelle 38 peuvent être fixées ensemble par la vis 37 qui est adaptée pour être introduite dans l'alésage axial 35 de la partie mâle 34 et pour être vissée dans un trou taraudé 42 agencé dans l'extrémité proximale 38 de la tige 39, axialement au-delà du logement conique 41. L'ouverture 36 dans la partie métaphysaire 33 permet l'introduction de la vis 37 dans l'alésage 35 et le trou 42.

Dans ce mode de réalisation, la longueur nécessaire pour la vis 37 est supérieure à celle nécessaire pour la vis 19 du mode de réalisation des figures 2 et 3.

Dans les trois formes de réalisation illustrées aux figures 2 à 5, la prothèse d'épaule est constituée de deux parties. En variante, il est possible de réaliser la partie métaphysaire 11 ou 33 et la tête 12 en deux parties distinctes pouvant être assemblées par des moyens adéquats, la prothèse étant alors réalisée en trois parties. Après pose de la tige humérale 9, 39 dans l'humérus fracturé, le chirurgien met en place la partie métaphysaire sur la tige, puis fixe la tête 12 sur la partie métaphysaire.

On comprend que, dans chaque forme de réalisation, la partie métaphysaire peut être rendue solidaire de la tête avec une orientation axiale quelconque de la tête autour de l'axe longitudinal de la partie ou tige métaphysaire.

Afin de pouvoir recevoir les éléments de la coiffe des muscles rotateurs, la partie sphérique de la tête 12 présente, à sa périphérie, des moyens de fixation de la coiffe, de préférence sous forme d'une pluralité de trous 20 à travers lesquels on pourra faire passer les fils de fixation de la coiffe sur la tête.

Pour les différents modes de réalisation possibles de la prothèse selon l'invention, le chirurgien met d'abord en place la tige humérale, puis fixe sur la tête 12, au moyen de fils, les muscles, en particulier la coiffe des rotateurs. Enfin, le chirurgien coiffe les parties d'os fracturé avec la tête 12, et emboîte la partie métaphysaire sur l'extrémité proximale de la tige. Si la tête 12 et la partie métaphysaire sont distinctes, la partie métaphysaire est d'abord mise en place, puis la tête fixée sur la partie métaphysaire.

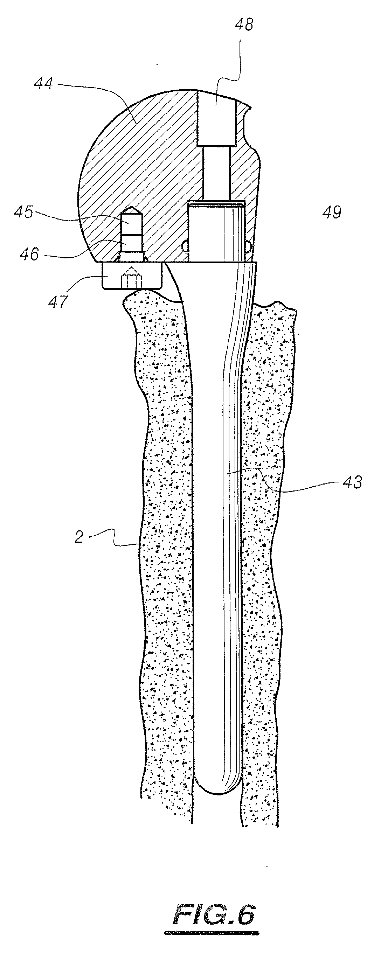

Pour le montage, on se réfère maintenant à la figure 6 qui représente les deux éléments d'une prothèse d'essai.

La prothèse d'essai comporte une tige diaphysaire d'essai 43 et une tête d'essai 44. La tête d'essai présente, à sa face inférieure, qui est perpendiculaire à l'axe géométrique de la prothèse, un trou taraudé 45 dans lequel peut être vissée une cale 46 dont la tête 47 s'applique contre cette face inférieure en restant en dehors de la prothèse d'essai. La tête 47 présente une épaisseur déterminée. Un orifice 48 permet la manipulation à l'aide d'un ancillaire.

Enfin la tête 44 présente des moyens 49 lui permettant d'être montée et démontée indifféremment sur la tige 43 de prothèse d'essai et sur les tiges 9 des prothèses selon l'invention.

Pour procéder au choix de la prothèse d'essai, on réalise, de préférence dans la phase pré-opératoire, des radiographies des deux bras gauche et droit. On superpose ensuite les deux radiographies, aussi exactement que possible. Le bord supérieur de la fracture de l'humérus sera alors pris comme point de référence et on choisira, à l'avance, une cale 46 d'une épaisseur permettant de ramener l'extrémité supérieure de la tête d'essai en coïncidence avec l'extrémité supérieure de la tête humérale du bras sain.

Pendant l'opération, après un alésage convenable du fût diaphysaire, le chirurgien met en place la prothèse d'essai, avec la cale 46 qui a été ainsi déterminée. Il enfonce la tige diaphysaire d'essai 43 dans le fût huméral et vérifie que, lorsque sa tête d'essai est mise en place, il retrouve la position correcte lorsque la cale est appliquée contre le bord supérieure de la fracture de l'humérus. En cas de besoin, il remplace la cale par une autre cale d'épaisseur différente.

Il connaît ainsi la longueur nécessaire de la prothèse et la longueur de la partie de l'autre prothèse qui doit émerger au-delà de la fracture humérale.

Il met ensuite en place, dans le fût diaphysaire, le bouchon habituel. Il monte la tête 44 de prothèse d'essai sur la partie diaphysaire définitive et introduit celle-ci dans le fût huméral jusqu'à obtenir à nouveau la butée de la pièce de butée contre le bord supérieur du trait de fracture humérale. La partie diaphysaire se trouve ainsi définitivement fixée. Il dépose ensuite la prothèse d'essai. Il procède ensuite à l'assemblage de la tête humérale définitive avec la coiffe des rotateurs en utilisant les fils de fixation, comme décrit dans la demande européenne EP-0940126. Il introduit ensuite la partie axiale mâle de la prothèse dans la partie axiale femelle correspondante, par exemple, le prolongement 18 dans l'orifice 13, d'une façon douce pour que la tête soit encore autorisée à tourner autour de la partie diaphysaire. Il peut ainsi laisser la tête être orientée par la coiffe des rotateurs dans la position de rétroversion anatomique. Il vérifie cette position. Si elle ne convient pas, il replace les fils de suture dans les trous 20 qui sont plus convenables.

Il procède ensuite à l'impaction et au vissage définitif de la tête sur la partie diaphysaire dans la position angulaire qui a ainsi été obtenue.

Il peut alors terminer l'intervention en refermant les plans anatomiques sus-jacents.

## Revendications

1. Prothèse d'épaule (5,32), comportant une tige humérale (9,39) destinée à être introduite dans le canal médullaire d'un humérus fracturé (2), et une tête (12), sensiblement hémisphérique susceptible d'être rendue solidaire de la tige humérale et adaptée pour venir se loger dans une glène de l'épaule, **caractérisée en ce que** la tête humérale (12), présente des moyens de fixation (20) des muscles de l'épaule; notamment des éléments de la coiffe, en plus de la fixation des fragments osseux ou tubérosités sur la tête, et que la tête peut être fixée sur la tige humérale dans un plan radial quelconque par rapport à l'axe de la tige humérale (9,39), après fixation de la tige (9, 39) dans l'humérus et fixation des muscles, notamment la coiffe des rotateurs sur la tête.

2. Prothèse d'épaule (5, 32), comportant une tige humérale (9, 39) destinée à être introduite dans le canal médullaire d'un humérus fracturé (2), n'ayant pas une forme de révolution, au moins vers son extrémité métaphysaire, pour être adaptée au canal médullaire, et une tête (12), sensiblement hémisphérique susceptible d'être rendue solidaire de la tige humérale et adaptée pour venir se loger dans une glène de l'épaule, **caractérisée en ce que** la tête humérale (12), peut être fixée sur la tige humérale dans un plan radial quelconque par rapport à l'axe de la tige humérale (9, 39), après fixation de la tige (9, 39) dans l'humérus et fixation des muscles, notamment la coiffe des rotateurs sur la tête.

3. Prothèse selon la revendication 2, **caractérisée en ce que** la tête humérale comprend des moyens de fixation (20) des muscles de l'épaule, notamment des éléments de la coiffe humérale, en plus de la fixation des fragments osseux ou tubérosités sur la tête.

4. Prothèse selon l'une des revendications 1 à 3, **caractérisée en ce que** ces moyens de fixations (20) sont répartis sur la périphérie de la tête humérale.

5. Prothèse selon l'une quelconque des revendications 2 à 4, **caractérisée en ce que** ces moyens de fixation sont des trous périphériques (20).

6. Prothèse selon l'une des revendications 1 à 5, **caractérisée en ce que** la tête humérale est solidaire d'une partie métaphysaire (11, 33) qui est susceptible d'être assemblée à la tige humérale dans une position angulaire quelconque.

7. Prothèse selon l'une des revendications 1 à 5, **caractérisée en ce que** la tête humérale forme une pièce distincte d'une partie métaphysaire susceptible d'être assemblée à la tête humérale dans une position angulaire quelconque.

8. Prothèse selon l'une des revendication 1 à 7, **caractérisée en ce que** les moyens de fixation sur la tige humérale comportent un assemblage d'éléments mâle et femelle (13, 18, 34, 38), permettant une fixation, après pose de la tête, dans n'importe quelle position angulaire relative.

9. Prothèse selon l'une des revendications 1 à 8, **caractérisée en ce que** l'assemblage de la tête humérale et de la tige humérale est réalisé au moyen d'un système mâle-femelle, notamment du type cône morse.

10. Prothèse selon la revendication 9, **caractérisée en ce qu'**une partie métaphysaire (11) solidaire de la tête délimite une partie femelle sous la forme d'une douille conique terminale (13) tandis que l'extrémité proximale associée de la tige (9) est constituée d'une partie conique mâle (18) complémentaire, adaptée pour être introduite dans la douille.

11. Prothèse selon la revendication 10, **caractérisée en ce que** les moyens de fixation comprennent une vis (19) adaptée pour être vissée dans un trou taraudé correspondant ménagé axialement dans la partie conique mâle (18), des moyens (16) étant prévus pour permettre l'introduction de la vis dans ledit trou taraudé.

12. Prothèse selon la revendication 11, **caractérisée en ce que** lesdits moyens consistent en un passage agencée dans la tête (12) axialement à la douille (13) afin d'autoriser la mise en place de la vis (19) dans celle-ci.

13. Prothèse selon la revendication 9, **caractérisée en ce que** ladite partie métaphysaire (33) délimite une partie mâle conique (34) et l'extrémité proximale de la tige (39) est constituée d'une partie femelle (38) formant un logement conique (41) adapté pour recevoir ladite partie mâle.

14. Prothèse selon la revendication 13, **caractérisée en ce que** lesdits moyens de fixation des parties mâle (34) et femelle (38) comportent une vis (37) adaptée pour être introduite dans un alésage axial (35) de la partie mâle conique (34), et pour être vissée dans un trou taraudé (42) agencé dans l'extrémité proximale (38) de la tige (39) axialement au-delà du logement conique (41).

15. Prothèse selon la revendication 14, **caractérisée en ce qu'**une ouverture (36) est formée dans la partie métaphysaire (33) axialement à l'alésage (35) de la partie mâle (34), afin de permettre l'introduction de la vis (37) dans cet alésage.

16. Prothèse selon l'une des revendications 9, 10 et 13, **caractérisée en ce qu'**une gorge circulaire (26) est agencée autour de la partie mâle conique (27) ainsi qu'un alésage transversal conique (28) dans la partie femelle correspondante (29), cet alésage débouchant dans la gorge en la tangentant sensiblement, et **en ce qu'**une goupille conique (31) de blocage desdites parties femelle et mâle est prévue pour être insérée dans l'alésage.

17. Prothèse selon l'une des revendications 6 à 16, **caractérisée en ce que** la partie métaphysaire (11, 33) est monobloc avec la tête hémisphérique (12), l'ensemble pouvant être solidarisé avec la tige (9, 39).

18. Prothèse selon l'une des revendications 1 à 17, **caractérisée en ce qu'**elle est constituée en trois parties pouvant être assemblées par des moyens appropriés, à savoir la tête hémisphérique (12), la partie métaphysaire et la tige humérale (9, 39).

19. Jeu de prothèses d'épaule, selon l'une des revendications 1 à 18, **caractérisé en ce qu'**il comporte une ou plusieurs tiges humérales et une ou plusieurs têtes humérales, les dimensions des tiges et/ou des têtes étant différentes.

20. Prothèse d'essai pour la pose de prothèses selon l'une quelconque des revendications 1 à 18, **caractérisée en ce qu'**elle possède une tige d'essai (43) correspondant à la tige d'essai de prothèse proprement dite, et une tête (44) correspondant à la tête de la prothèse proprement dite, ladite tête portant une cale (46) d'une épaisseur déterminée susceptible de venir en contact avec une extrémité de fût huméral pour la détermination de la taille de la prothèse proprement dite.

21. Prothèse d'essai selon la revendication 20, **caractérisée en ce que** la tête d'essai (44) est amovible et peut être montée, et démontée, sur la tige d'essai (43) et sur les tiges de prothèse (9,39).

22. Procédé de mise en place d'une prothèse d'épaule (5,32) comprenant une tige humérale (9, 39) destinée à être introduite dans le canal médullaire d'un humérus fracturé (2') et une tête (12) sensiblement hémisphérique susceptible d'être rendue solidaire de la tige humérale et adaptée pour venir se loger dans une glène de l'épaule, ladite tête pouvant être fixée sur la tige humérale dans un plan radial quelconque par rapport à l'axe de la tige humérale (9,39), comprenant les étapes de positionnement de la tige humérale à la hauteur adéquate dans le canal médullaire, le scellement de ladite tige dans ledit canal, la fixation des muscles de l'épaule sur la tête, le positionnement des fragments osseux sur la tête, le montage de la tête sur la tige scellée, le réglage de rétroversion de la tête en rotation par rapport à la tige, puis la fixation définitive de la tête sur la tige.

23. Procédé selon la revendication 20 dans lequel la prothèse d'épaule est une prothèse selon l'une quelconque des revendications 1 à 16.
